# FASCICULE DE BREVET EUROPEEN

(11) **EP 0 968 682 B1**
(45) Date de publication et mention de la délivrance du brevet: **04.02.2004**
(21) Numéro de dépôt: 99401604.6
(22) Date de dépôt: 28.06.1999
(51) Int. Cl.: A61B 5/15, A61M 5/32

(54) **Dispositif de prélèvement sanguin à tube sous vide**
Blutentnahmevorrichtung für ein vakuum evakuiertes Röhrchen
Blood sampling device for a vacuum evacuated tube

(30) Priorité: 03.07.1998 FR 9808551
(43) Date de publication de la demande: 05.01.2000
(73) Titulaire: Altair, 14370 Argences (FR)
(72) Inventeur: Arruego, Daniel Pierre Henri, 14370 Chicheboville (FR); Lloze, Alain Bernard, 14210 Amaye sur Orne (FR)
(74) Mandataire: Lemoine, Robert

(56) Documents cités:
- EP-A- 0 539 634
- EP-A- 0 553 024
- WO-A-89/04141

## Description

La présente invention concerne un dispositif de prélèvement sanguin pour introduire un échantillon de sang dans un tube sous vide comportant un bouchon transperçable, ce dispositif comprenant :
- un corps tubulaire comportant une paroi latérale pourvue d'au moins une fente longitudinale se terminant par des élargissements postérieur et antérieur, et une paroi transversale antérieure pourvue d'un orifice ; et
- une canule ayant une partie antérieure et une partie postérieure s'étendant de part et d'autre d'un coulisseau logé dans le corps tubulaire et coopérant avec la fente longitudinale, le coulisseau étant déplaçable entre une position extrême postérieure dans laquelle il est verrouillé dans l'élargissement postérieur de la fente tandis que les parties antérieure et postérieure de la canule sont situées à l'intérieur du corps tubulaire, et une position extrême antérieure dans laquelle il est verrouillé dans l'élargissement antérieur de la fente tandis que la partie antérieure de la canule traverse l'orifice de la paroi transversale du corps tubulaire et fait saillie hors de celui-ci en vue de son implantation dans une zone de prélèvement et de l'introduction d'un échantillon de sang dans le tube sous vide, après la mise en place de ce dernier dans le corps tubulaire et le perçage du bouchon par l'extrémité postérieure de la canule.

Les dispositifs de prélèvement sanguin actuels sont utilisables plusieurs fois, ce qui peut avoir de graves conséquences pour la santé des personnes soumises aux prélèvements.

La présente invention se propose d'empêcher une telle réutilisation et, pour ce faire, elle a pour objet un dispositif de prélèvement sanguin se caractérisant en ce qu'il comprend des moyens de sécurité pour immobiliser définitivement le coulisseau dans sa position extrême postérieure lorsque celui-ci est ramené dans cette position après avoir été verrouillé dans l'élargissement antérieur de la fente longitudinale.

Grâce à ces moyens de sécurité, le dispositif de prélèvement conforme à l'invention ne peut plus être réutilisé s'il a servi une fois. Les risques de contamination des personnes soumises aux prélèvements sont donc éliminés en totalité.

Par ailleurs, comme la canule est en totalité à l' intérieur du corps tubulaire lorsque le coulisseau est dans sa position extrême postérieure, les personnes susceptibles de manipuler les dispositifs de prélèvement sont protégées contre les piqûres accidentelles et par conséquent contre les risques de contamination.

Selon l'invention, les moyens de sécurité comprennent des organes mobiles situés sur le coulisseau et des organes fixes situés sur la face intérieure de la paroi transversale antérieure du corps tubulaire.

Les organes mobiles comprennent:
- un arceau élastique comportant au moins un téton déplaçable entre une position rétractée dans laquelle il est entièrement à l'intérieur du corps tubulaire et une position avancée dans laquelle il est au moins partiellement à l'extérieur du corps tubulaire et fait saillie dans une encoche latérale ménagée dans l'élargissement postérieur de la-fente longitudinale, et
- un crochet déplaçable entre une position active dans laquelle il est en prise avec l'arceau et retient le téton dans sa position rétractée, et une position inactive dans laquelle il est séparé de l'arceau et autorise le téton à venir dans sa position avancée lorsque le coulisseau est ramené dans sa position extrême postérieure.

Quant aux organes fixes, ils peuvent comprendre un doigt destiné à déplacer le crochet de sa position active à sa position inactive lorsque le coulisseau vient dans sa position extrême antérieure.

Ainsi, lorsque le coulisseau arrive dans sa position extrême antérieure, le doigt porté par la paroi transversale du corps tubulaire déplace automatiquement le crochet de sa position active à sa position inactive. L'arceau, qui est élastique, se détend alors vers l'extérieur dès que le crochet le libère. Par suite, lorsque le coulisseau est ramené dans sa position extrême postérieure, le téton vient dans sa position avancée à l'intérieur de l'encoche latérale de l'élargissement postérieur de la fente longitudinale et immobilise définitivement le coulisseau.

Avantageusement, le coulisseau peut comprendre un organe de commande porté par un deuxième arceau élastique situé derrière l'arceau portant le téton, l'organe de commande étant sollicité radialement vers l'extérieur par le deuxième arceau et comportant un poussoir faisant saillie hors de la fente longitudinale, une embase dont l'étendue correspond à celle des élargissements de la fente longitudinale, et une zone de guidage située entre le poussoir et l'embase et dont la largeur correspond à celle de la partie de la fente longitudinale qui est située entre les élargissements antérieur et postérieur.

Etant sollicité radialement vers l'extérieur par le second arceau, l'organe de commande verrouille le coulisseau lorsque celui-ci est dans sa position extrême postérieure d'origine ou dans sa position extrême antérieure.

- L'embase de l'organe de commande se trouve en effet chaque fois dans l'un des élargissements de la fente longitudinale et empêche un déplacement longitudinal du coulisseau le long de celle-ci.

Pour pouvoir effectuer un tel déplacement, il convient d'abord d'exercer une pression sur le poussoir pour amener la zone de guidage devant la fente longitudinale, puis d'exercer sur le poussoir une poussée longitudinale dans le sens voulu.

De préférence, l'embase peut comporter deux pattes transversales tournées l'une vers l'autre et ménageant entre elles un logement dans lequel le deuxième arceau est encliqueté.

Lors de la fabrication du dispositif de prélèvement conforme à l'invention, on introduit le coulisseau, sans l'organe de commande, dans le corps tubulaire, l'introduction étant bien entendu réalisée du côté de l'extrémité ouverte de celui-ci.

Ce n'est en fait qu'après avoir disposé le coulisseau dans sa position extrême postérieure que l'on encliquette l'embase de l'organe de commande sur le second arceau.

Une telle opération est facile à réaliser, surtout lorsque le matériau constituant le coulisseau présente une certaine élasticité. Il suffit en effet d'exercer une pression sur l'organe de commande pour que les pattes transversales de l'embase s'écartent l'une de l'autre et permettent au deuxième arceau de venir se loger dans le logement ménagé entre elles.

Selon une mode de réalisation avantageux de l'invention, le poussoir possède une surface de préhension dont l' étendue est déterminée pour qu'il recouvre l'élargissement postérieur de la fente longitudinale du corps tubulaire ainsi que l' encoche latérale de cet élargissement, lorsque le coulisseau est dans sa position extrême postérieure.

L'étendue de la surface de préhension est choisie pour que celle-ci recouvre le téton lorsque le coulisseau est dans sa position extrême postérieure et interdise le déverrouillage frauduleux de ce dernier à l'aide d'un outil quelconque.

De préférence, le coulisseau peut comprendre un troisième arceau situé devant l'arceau portant le téton et dont les dimensions extérieures correspondent, au jeu de coulissement près, aux dimensions intérieures du corps tubulaire.

Grâce à ce troisième arceau, le coulisseau peut être guidé de façon très sûre, ce qui évite son coincement et tout risque de défaillance du dispositif de prélèvement.

Selon une première variante de réalisation, la canule est contrecoudée, ses parties antérieure et postérieure étant parallèles entre elles, la première étant adjacente à la paroi latérale du corps tubulaire tandis que la seconde est coaxiale ou pratiquement coaxiale avec ce dernier.

Comme la partie antérieure de la canule est adjacente à la paroi latérale du corps tubulaire, son implantation dans la zone de prélèvement peut être réalisée en plaçant le dispositif de prélèvement dans une position pratiquement rasante par rapport à la zone de prélèvement, ce qui facilite la tâche du praticien.

Par ailleurs, comme la partie postérieure de la canule est coaxiale ou pratiquement coaxiale avec l'axe du corps tubulaire, elle transperce à coup sûr le bouchon du tube sous vide utilisé.

Selon une deuxième variante de réalisation, la canule est rectiligne et s'étend parallèlement au corps tubulaire, entre l'axe et la périphérie de celui-ci.

Cette deuxième variante a l'avantage d'être moins coûteuse à réaliser tout en permettant de placer le dispositif de prélèvement dans une position plus rasante que dans le cas des dispositifs actuels et de percer le bouchon du tube sous vide quand même avec certitude.

Selon une autre caractéristique de l'invention, le corps tubulaire comporte à son extrémité postérieure ouverte, une collerette externe dont la périphérie est conformée pour constituer une surface d'appui instable.

- Il est ainsi impossible de disposer le dispositif de prélèvement verticalement sur une table ou toute autre surface d'appui. Les risques pour qu'une personne se pique dans le cas où le coulisseau n'aurait pas été ramené dans sa position extrême postérieure sont donc très réduits.

Trois modes d'exécution de la présente invention seront décrits ci-après à titre d'exemples nullement limitatifs en référence aux dessins annexés dans lesquels :
- la figure 1 est une vue en coupe longitudinale d'un premier dispositif de prélèvement conforme à l'invention, le coulisseau étant dans sa position extrême postérieure d'origine ;
- la figure 2 est une vue en coupe selon la ligne II-II de la figure 3, le coulisseau étant entre ses positions extrêmes postérieure et antérieure ;
- la figure 3 est une vue de dessus du dispositif représenté sur la figure 2 ;
- la figure 4 est une vue en coupe selon la ligne IV-IV de la figure 1 ;
- la figure 5 est une vue selon la ligne V-V de la figure 3 ;
- la figure 6 est une vue en perspective du coulisseau, le poussoir étant désolidarisé de son arceau ;
- la figure 7 est une vue en coupe longitudinale du dispositif de prélèvement, le coulisseau étant dans sa position extrême antérieure ;
- la figure 8 est une vue en coupe selon la ligne VIII-VIII de la figure 7 ;
- la figure 9 est une vue en coupe longitudinale du dispositif de prélèvement, le coulisseau ayant été ramené dans sa position extrême postérieure ;
- la figure 10 est une vue en coupe selon la ligne X-X de la figure 9 ;
- la figure 11 est une vue en bout d'un deuxième dispositif de prélèvement conforme à l'invention, cette vue étant prise du côté de la partie antérieure de la canule ; et
- la figure 12 est une vue en coupe longitudinale d'un troisième dispositif de prélèvement conforme à l'invention.

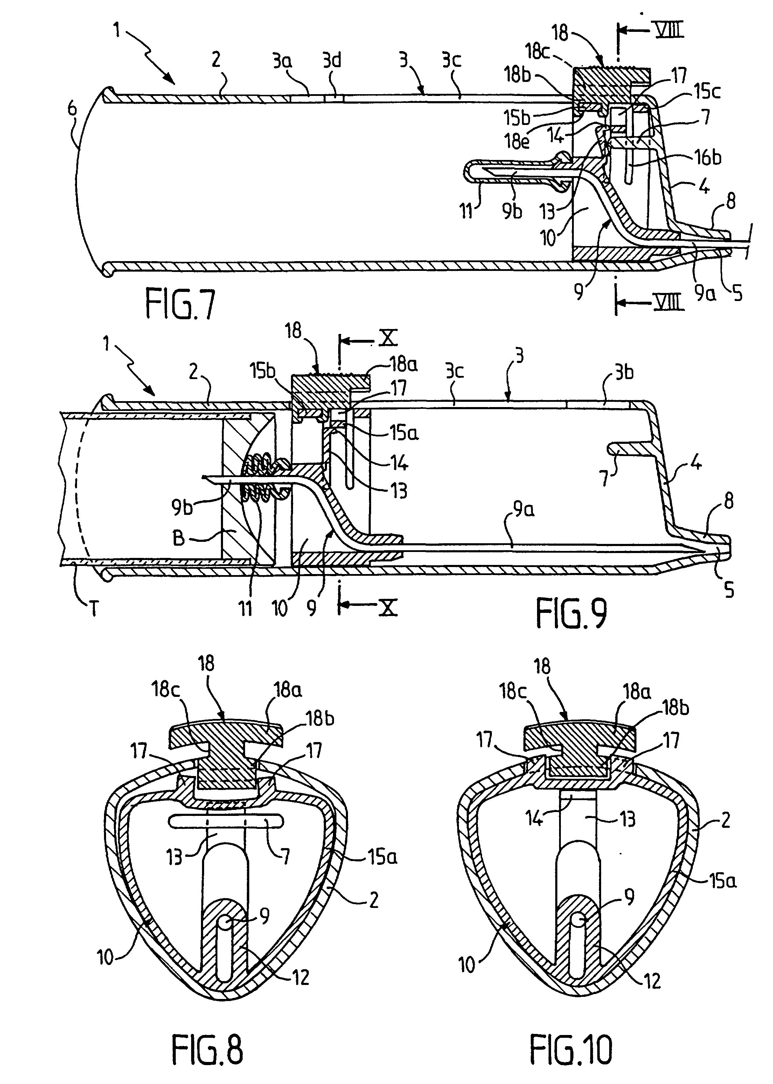

Les dispositifs de prélèvement sanguin représentés sur les dessins sont destinés à introduire un échantillon de sang dans des tubes transparents tels que le tube T représenté partiellement sur la figure 9.

Ces tubes qui sont maintenus sous vide par des bouchons transperçables B, par exemple en caoutchouc, sont de conception classique et ne font évidemment pas partie de l'invention.

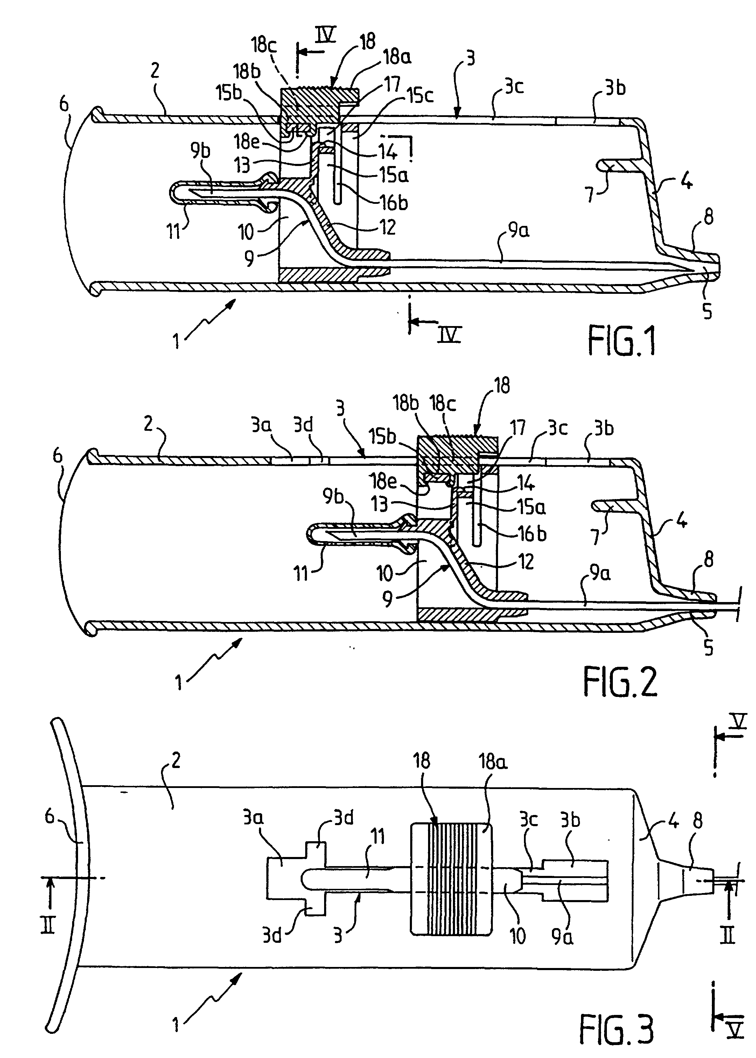

Le dispositif de prélèvement représenté sur les figures 1 à 10 comprend un corps tubulaire 1 comportant une paroi latérale 2 pourvue d'une fente longitudinale 3 et une paroi transversale antérieure 4 pourvue d'un orifice 5.

Le corps tubulaire 1 est de préférence réalisé en matière plastique transparente. Sa paroi latérale 2 a en coupe transversale une forme triangulaire tandis que son extrémité postérieure ouverte comporte une collerette externe 6 conformée pour l'empêcher de tenir debout lorsqu'elle est directement posée sur un support tel qu'une table ou une paillasse.

Comme le montrent les figures 3 et 5, la collerette 6 possède une forme elliptique, a une épaisseur constante et ménage une cuvette concave entourant l'extrémité ouverte du corps tubulaire 1. Grâce à sa forme elliptique, la collerette 6 empêche le dispositif de prélèvement de rouler sur un support et de tomber.

La fente longitudinale 3 comporte un élargissement terminal postérieur 3a et un élargissement terminal antérieur 3b reliés par une partie intermédiaire 3c ayant une largeur constante.

L'élargissement postérieur 3a a la forme d'un T dont la branche transversale est constituée par deux encoches rectangulaires 3d situées du côté de l'élargissement antérieur 3b.

Quant à l'élargissement antérieur 3b, il est rectangulaire et a une largeur égale à celle de la branche longitudinale du T constituant l'élargissement postérieur 3a.

Par ailleurs, la paroi transversale antérieure 4 du corps tubulaire 1 est pourvue sur sa face interne d'un doigt 7 dont le rôle sera indiqué ci-après et sur sa face externe d'une saillie tubulaire 8 dont le conduit interne constitue l'orifice 5.

On notera ici que l'axe de l'orifice 5 est parallèle à l'axe longitudinal du corps tubulaire 1.

Le dispositif de prélèvement comprend également une canule 9 s'étendant de part et d'autre d'un coulisseau 10 logé dans le corps tubulaire 1 et coopérant avec la fente longitudinale 3.

La canule 9 comporte une partie antérieure 9a destinée à être implantée dans une veine en vue du prélèvement d'un échantillon de sang et une partie postérieure 9b destinée à transpercer le bouchon B d'un tube sous vide T prévu pour recevoir l'échantillon de sang prélevé.

Cette canule est contrecoudée et ses parties antérieures 9a et postérieure 9b sont parallèles entre elles, la première étant adjacente à la paroi latérale 2 du corps tubulaire 1, tandis que la seconde est coaxiale avec ce dernier.

En se référant aux dessins, on remarquera que la partie postérieure 9b de la canule est recouverte d'un capuchon de protection 11 en caoutchouc.

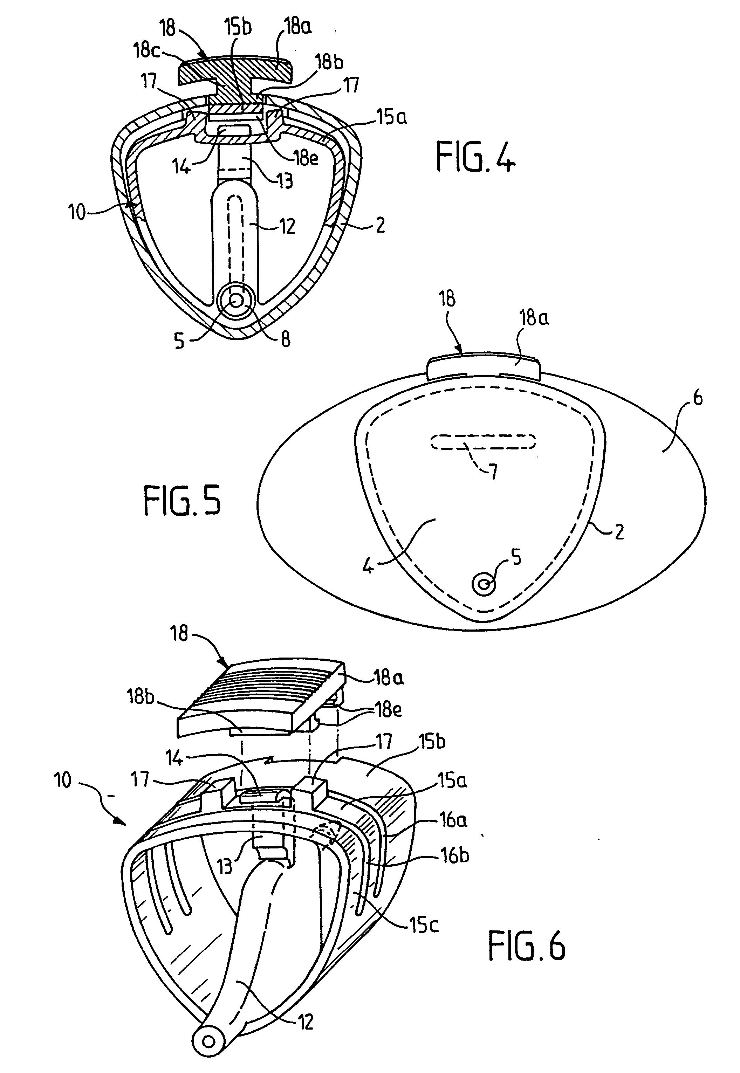

Le coulisseau 10 est déplaçable dans le corps tubulaire 1 entre une position extrême postérieure (visible sur les figures 1 et 4) dans laquelle il est verrouillé dans l'élargissement postérieur 3a, 3d de la fente 3 et une position extrême antérieure (visible sur la figure 7) dans laquelle il est verrouillé dans l'élargissement antérieur 3b de la fente 3.

Lorsque le coulisseau 10 est dans sa position extrême postérieure, les parties antérieure 9a et postérieure 9b de la canule 9 sont toutes les deux situées à l' intérieur du corps tubulaire. En revanche, lorsqu'il est dans sa position extrême antérieure, la partie antérieure 9a de la canule fait saillie hors du corps tubulaire 1 au niveau de l'orifice 5 et peut ainsi être implanté dans une veine.

Il convient bien entendu d'introduire un tube T dans le corps tubulaire 1 afin que la partie postérieure 9b de la canule transperce le bouchon B et que la dépression régnant dans le tube assure l'introduction dans ce dernier d'un échantillon de sang provenant de la veine dans laquelle la partie antérieure 9a de la canule a été implantée.

Le coulisseau 10 comprend un corps tubulaire dont la section est pratiquement la même que celle du corps tubulaire 1 et qui est particulièrement bien visible sur la figure 6.

Une protubérance 12 s'étendant axialement à l'intérieur du coulisseau occupe un peu plus de la moitié inférieure de celui-ci. Elle entoure la partie contrecoudée de la canule 9 et rend cette dernière solidaire du coulisseau. En outre, elle est pourvue d'un crochet 13 dont le rôle sera décrit ci-après, ce crochet faisant saillie vers le haut au niveau de la partie la plus élevée de la protubérance et se terminant par une languette 14 tournée vers la paroi transversale antérieure 4 du corps tubulaire 1.

Approximativement la moitié supérieure de la paroi latérale du coulisseau 10 est divisée en trois arceaux 15a, 15b et 15c par deux fentes transversales 16a, 16b.

L'arceau 15a, désigné par premier arceau ou arceau intermédiaire, porte sur sa face externe deux tétons 17 dont l'étendue et la distance correspondent à celles des encoches rectangulaires 3d de l'élargissement postérieur 3a de la fente 3.

Le crochet 13 dont il a été question ci-dessus est en prise avec l'arceau 15a et empêche les tétons 17 de pénétrer dans les encoches 3d lorsque le coulisseau est dans sa position extrême postérieure d'origine représentée sur la figure 1.

L'arceau 15b, désigné par deuxième arceau ou arceau postérieur, est situé du côté opposé à la paroi transversale antérieure 4 du corps tubulaire 1 et supporte un organe de commande 18 à l'aide duquel un utilisateur peut déplacer le coulisseau 10 le long de la fente 3.

L'organe de commande 18 comporte un poussoir 18a faisant saillie hors de la fente 3, une embase 18b dont l'étendue correspond à celle des élargissements antérieur 3b et postérieur 3a ( à l' exclusion des encoches 3d) de la fente, et une zone de guidage 18c située entre le poussoir 18a et l'embase 18b et dont la largeur correspond à celle de la partie intermédiaire 3c de la fente 3.

Le poussoir 18a possède une surface de préhension rectangulaire qui, lorsque le coulisseau 10 est dans sa position extrême postérieure, recouvre entièrement l'élargissement postérieur 3a et ses encoches 3d et empêche ainsi tout accès aux tétons 17.

Par ailleurs, l'embase 18b comporte sur sa face inférieure deux pattes transversales 18e tournées l'une vers l'autre et ménageant entre elles un logement dans lequel l'arceau postérieur 15b est encliqueté.

L'arceau 15c, désigné par troisième arceau ou arceau antérieur, est prévu pour faciliter le déplacement du coulisseau 10 à l'intérieur du corps tubulaire 2. A cet effet, ses dimensions extérieures correspondent, au jeu de coulissement près, aux dimensions intérieures du corps tubulaire 2.

On va maintenant décrire les opérations qu'il convient d'effectuer pour utiliser le dispositif de prélèvement conforme à l'invention.

On précisera tout d'abord que ce dispositif est livré alors que le coulisseau 10 est dans sa position extrême postérieure représentée sur la figure 1.

Dans cette position, le crochet 13 est en prise avec l'arceau intermédiaire 15a et empêche les tétons 17 de faire saillie dans les encoches 3d de l' élargissement postérieur 3a de la fente longitudinale du corps tubulaire 1.

En revanche, l'embase 18b fait saillie dans l'élargissement postérieur 3a et s'oppose à ce que le coulisseau 10 soit déplacé longitudinalement en direction de la paroi antérieure 4 du corps tubulaire.

Pour déplacer le coulisseau vers cette paroi, il convient en fait d'exercer une pression sur le poussoir 18a afin d'amener la zone de guidage 18c à la hauteur de la fente longitudinale 3 et de pousser ensuite le poussoir 18a en direction de la paroi transversale 4.

Pendant ce temps, la partie antérieure 9a de la canule s'avance dans l'orifice 5 et fait progressivement saillie à l'extérieur du corps tubulaire.

En se référant à la figure 2, on remarquera que les tétons 17 et l'embase 18b sont à l'intérieur du corps tubulaire et que le crochet 13 est toujours en prise avec l'arceau 15a.

Puis, lorsque le coulisseau 10 arrive dans sa position extrême antérieure, l'embase 18b fait saillie élastiquement dans l'élargissement antérieur 3b de la fente longitudinale 3 tandis que le doigt 7 prévu sur la face interne de la paroi transversale 4 pousse le crochet 13 vers l'arrière et le désolidarise de l'arceau 15a.

En raison de l'élasticité du matériau utilisé pour réaliser le coulisseau 10, les tétons 17 viennent s'appuyer contre la surface interne de la paroi longitudinale 2 du corps tubulaire 1, comme représenté sur la figure 8.

Comme l'embase 18b est situé dans l'élargissement antérieur 3b, le coulisseau 10 est verrouillé de sorte que le dispositif de prélèvement peut être utilisé pour introduire un échantillon de sang dans un tube T.

Après la réalisation du prélèvement, il convient d'exercer à nouveau une pression sur le poussoir 18a afin d'amener la zone de guidage 18c à la hauteur de la fente longitudinale 3 et de pousser ensuite le poussoir 18a de façon à l'éloigner de la paroi transversale 4.

Puis, lorsque le coulisseau parvient dans sa position extrême postérieure, l'embase 18b fait saillie élastiquement dans l'élargissement postérieur 3a.

Simultanément, les tétons 17 viennent élastiquement dans les encoches 3d de l'élargissement 3a, comme représenté sur la figure 10 tandis que la languette 14 du crochet 13 vient élastiquement sous l'arceau 15a et s'oppose à un abaissement de celui-ci, comme représenté sur la figure 9.

Il est maintenant impossible de ramener le coulisseau 10 dans sa position extrême antérieure et d'effectuer un nouveau prélèvement.

En effet, le fait d'exercer une pression sur le poussoir 18a ne permet pas d'extraire les tétons 17 des encoches 3d. Par ailleurs, comme le poussoir recouvre entièrement les encoches 3d, il est impossible d'accéder aux tétons à l'aide d'un outil quelconque et donc de les extraire frauduleusement desdites encoches.

Il ressort de ce qui précède que le dispositif de prélèvement conforme à l'invention est conçu pour n'être utilisé qu'une seule fois, ce qui évite les risques de contamination des patients.

Par ailleurs, comme la canule 9 est entièrement contenue dans le corps tubulaire 1 lorsque le coulisseau 10 est ramené dans sa position extrême postérieure, les risques pour que la personne chargée du prélèvement sanguin se blesse accidentellement sont éliminés.

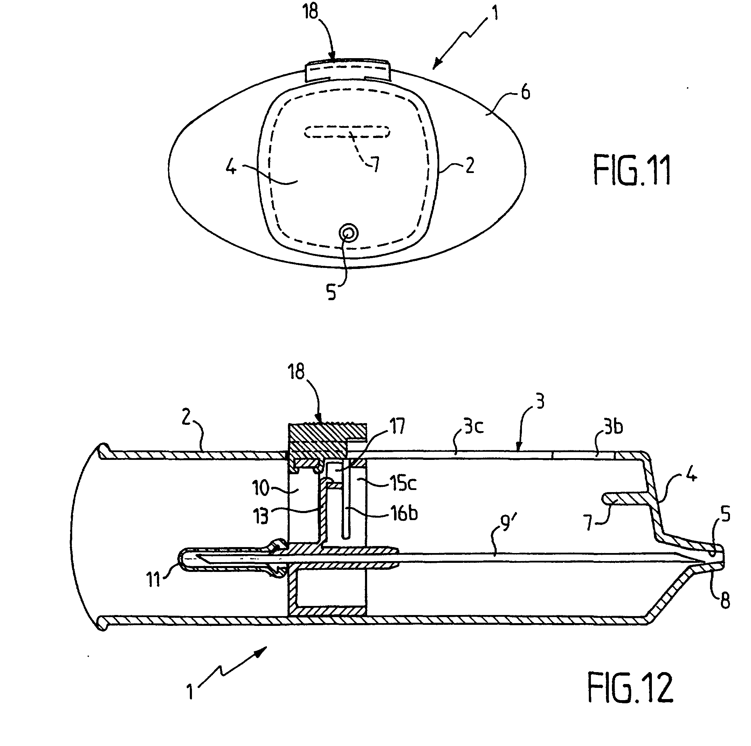

En se référant maintenant à la figure 11, on remarquera que le dispositif de prélèvement visible sur cette figure possède un corps tubulaire 1 ayant une section pratiquement carrée et des angles arrondis.

Il va de soi que l'on ne sortirait pas du cadre de la présente invention si le corps tubulaire avait une section circulaire.

En se référant enfin à la figure 12, on remarquera que le dispositif de prélèvement représenté comporte une canule rectiligne 9' s'étendant parallèlement au corps tubulaire 1, cette canule étant située dans une position intermédiaire entre l'axe longitudinal du corps tubulaire et la paroi longitudinale de celui-ci.

## Revendications

1. Dispositif de prélèvement sanguin pour introduire un échantillon de sang dans un tube sous vide (T) comportant un bouchon (B) transperçable, dispositif comprenant :
- un corps tubulaire (1) comportant une paroi latérale (2) pourvue d'au moins une fente longitudinale (3) se terminant par des élargissements postérieur (3a) et antérieur (3b), et une paroi transversale antérieure (4) pourvue d'un orifice (5) ;
- une canule (9 ; 9') ayant une partie antérieure (9a) et une partie postérieure (9b) ;
- un coulisseau (10) portant la canule (9 ; 9') et coopérant avec la fente longitudinale (3), le coulisseau étant déplaçable dans le corps tubulaire (1) entre une position extrême postérieure dans laquelle les parties antérieure (9a) et postérieure (9b) de la canule (9 ; 9') sont situées à l'intérieur du corps tubulaire (1), et une position extrême antérieure dans laquelle la partie antérieure (9a) de la canule (9 ; 9') traverse l'orifice (5) de la paroi transversale (4) du corps tubulaire (1) et fait saillie hors de celui-ci en vue du prélèvement d'un échantillon de sang et de l'introduction de celui-ci dans le tube sous vide (T) ; et
- des moyens de sécurité (7, 13, 14, 15a, 17) pour immobiliser définitivement le coulisseau (10) dans sa position extrême postérieure lorsque celui-ci est ramené dans cette position après la réalisation d'un prélèvement, les moyens de sécurité comprenant des organes mobi les (13, 14, 15a, 17) situés sur le coulisseau (10) et des organes fixes (7) situés sur le corps tubulaire (1), **caractérisé en ce que** les organes mobiles comprennent :
- un arceau élastique (15a) comportant au moins un téton (17) déplaçable entre une position rétractée dans laquelle il est entièrement à l'intérieur du corps tubulaire (1) et une position avancée dans laquelle il est au moins partiellement à l'extérieur du corps tubulaire (1) et fait saillie dans une encoche latérale (3d) ménagée dans l'élargissement postérieur (3a) de la fente longitudinale (3) , et
- un crochet (13, 14) déplaçable entre une position active dans laquelle il est en prise avec l'arceau (15a) et retient le téton (17) dans sa position rétractée, et une position inactive dans laquelle il est séparé de l'arceau (15a) et autorise le téton (17) à venir dans sa position avancée lorsque le coulisseau (10) est ramené dans sa position extrême postérieure.

2. Dispositif selon la revendication 1, **caractérisé en ce que** les organes fixes sont situés sur la face intérieure de la paroi transversale antérieure (4) du corps tubulaire (1).

3. Dispositif selon la revendication 2, **caractérisé en ce que** les organes fixes comprennent un doigt (7) destiné à déplacer le crochet (13, 14) de sa position active à sa position inactive lorsque le coulisseau (10) vient dans sa position extrême antérieure.

4. Dispositif selon l'une quelconque des revendications 1 à 3, **caractérisé en ce que** le coulisseau (10) comprend un organe de commande (18) porté par un deuxième arceau élastique (15b) situé derrière l'arceau (15a) portant le téton (17), l'organe de commande étant sollicité radialement vers l'extérieur par le deuxième arceau (15b) et comportant un poussoir (18a) faisant saillie hors de la fente longitudinale (3), une embase (18b) dont l'étendue correspond à celle des élargissements (3a, 3b) de la fente longitudinale (3), et une zone de guidage (18c) située entre le poussoir (18a) et l'embase (18b) et dont la largeur correspond à celle de la partie de la fente longitudinale (3) qui est située entre les élargissements antérieur (3b) et postérieur (3a).

5. Dispositif selon la revendication 4, **caractérisé en ce que** l'embase (18b) comporte deux pattes transversales (18e) tournées l'une vers l'autre et ménageant entre elles un logement dans lequel le deuxième arceau (15b) est encliqueté.

6. Dispositif selon la revendication 4 ou 5, **caractérisé en ce que** le poussoir (18a) possède une surface de préhension dont l'étendue est déterminée pour qu'il recouvre l'élargissement postérieur (3a) de la fente longitudinale (3) du corps tubulaire (1) ainsi que l'encoche latérale (3d) de cet élargissement, lorsque le coulisseau (10) est dans sa position extrême postérieure.

7. Dispositif selon l'une quelconque des revendications 1 à 6, **caractérisé en ce que** le coulisseau (10) comprend un troisième arceau (15c) situé devant l'arceau (15a) portant le téton (17) et dont les dimensions extérieures correspondent, au jeu de coulissement près, aux dimensions intérieures du corps tubulaire (1).

8. Dispositif selon l'une quelconque des revendications 1 à 7, **caractérisé en ce que** la canule (9) est contrecoudée, ses parties antérieure (9a) et postérieure (9b) étant parallèles entre elles, la première étant adjacente à la paroi latérale (2) du corps tubulaire (1) tandis que la seconde est coaxiale ou pratiquement coaxiale avec ce dernier.

9. Dispositif selon l'une quelconque des revendications 1 à 7, **caractérisé en ce que** la canule (9') est rectiligne et s'étend parallèlement au corps tubulaire (1), entre l'axe et la périphérie de celui-ci.

10. Dispositif selon l'une quelconque des revendications précédentes, **caractérisé en ce que** le corps tubulaire (1) comporte à son extrémité postérieure ouverte, une collerette externe (6) dont la périphérie est conformée pour constituer une surface d'appui instable.

## Claims

1. A blood sampling device for introducing a blood sample into a vacuum tube (T) comprising a pierceable stopper (B), the device including:
- a tubular body (1) comprising a side wall (2) provided with at least one longitudinal slot (3) ending in rear (3a) and front (3b) enlargements and a front transverse wall (4) provided with an orifice (5);
- a cannula (9; 9') having a front part (9a) and a rear part (9b);
- a slide (10) carrying the cannula (9; 9') and cooperating with the longitudinal slot (3), the slide being displaceable in the tubular body (1) between a rear end position in which the front (9a) and rear (9b) parts of the cannula (9; 9') are located inside the tubular body (1), and a front end position in which the front part (9a) of the cannula (9; 9') passes through the orifice (5) of the transverse wall (4) of the tubular body (1) and protrudes from the latter for the purpose of taking a blood sample and introducing the latter into the vacuum tube (T); and
- safety means (7, 13, 14, 15a, 17) for immobilizing the slide (10) definitively in its rear end position when it has been brought back into this position after a sample has been taken, the safety means including movable members (13, 14, 15a, 17) located on the slide (10) and fixed members (7) located on the tubular body (1), **characterized in that** the movable members include:
- an elastic bow (15a) comprising at least one stud (17) displaceable between a retracted position in which it is entirely inside the tubular body (1) and an advanced position in which it is at least partially outside the tubular body (1) and projects into a lateral notch (3d) formed in the rear enlargement (3a) of the longitudinal slot (3); and
- a hook (13, 14) displaceable between an active position in which it engages with the bow (15a) and retains the stud (17) in its retracted position, and an inactive position in which it is separated from the bow (15a) and allows the stud (17) to come into its advanced position when the slide (10) is brought back into its rear end position.

2. The device according to claim 1, **characterized in that** the fixed members are located on the inner face of the front transverse wall (4) of the tubular body (1).

3. The device according to claim 2, **characterized in that** the fixed members include a finger (7) designed to displace the hook (13, 14) from its active position to its inactive position when the slide (10) comes into its front end position.

4. The device according to any one of claims 1 to 3, **characterized in that** the slide (10) includes a control member (18) carried by a second elastic bow (15b) located behind the bow (15a) bearing the stud (17), the control member being urged radially outwards by the second bow (15b) and comprising a pusher (18a) projecting outside the longitudinal slot (3), a base (18b) the extent of which corresponds to that of the enlargements (3a, 3b) of the longitudinal slot (3), and a guide zone (18c) located between the pusher (18a) and the base (18b) and the width of which corresponds to that of the part of the longitudinal slot (3) that is located between the front (3b) and rear (3a) enlargements.

5. The device according to claim 4, **characterized in that** the base (18b) comprises two transverse tabs (18e) facing one another and forming between them a housing into which the second bow (15b) is snap-fitted.

6. The device according to claim 4 or 5, **characterized in that** the pusher (18a) has a grip surface the extent of which is determined so that it covers the rear enlargement (3a) of the longitudinal slot (3) of the tubular body (1) as well as the lateral notch (3d) of this enlargement, when the slide (10) is in its rear end position.

7. The device according to any one of claims 1 to 6, **characterized in that** the slide (10) includes a third bow (15c) located in front of the bow (15a) bearing the stud (17) and the external dimensions of which correspond, except for the sliding clearance, to the internal dimensions of the tubular body (1).

8. The device according to any one of claims 1 to 7, **characterized in that** the cannula (9) is cranked, with its front (9a) and rear (9b) parts parallel to one another, the first part being adjacent to the side wall (2) of the tubular body (1) while the second part is coaxial, or practically coaxial, with the latter.

9. The device according to any one of claims 1 to 7, **characterized in that** the cannula (9') is rectilinear and extends parallel to the tubular body (1), between the axis and the periphery thereof.

10. The device according to any one of the preceding claims, **characterized in that** the tubular body (1) comprises, at its open rear end, an external flange (6) the periphery of which is shaped to form an unstable bearing surface.

## Patentansprüche

1. Blutentnahmevorrichtung für ein Einleiten einer Blutprobe in ein Vakuumröhrchen (T), das einen durchstechbaren Pfropfen (B) aufweist, wobei die Vorrichtung besteht aus:
- einem rohrförmigen Körper (1), der eine Seitenwand (2) aufweist, die mit wenigstens einem Längsschlitz (3) versehen ist, welcher in hinteren (3a) und vorderen (3b) Erweiterungen endet, und eine vordere Querwand (4), die mit einer Öffnung (5) versehen ist;
- einer Kanüle (9; 9'), die einen vorderen Bereich (9a) und einen hinteren Bereich (9b) hat;
- einem Schieber (10), der die Kanüle (9; 9') trägt und mit dem Längsschlitz (3) zusammenwirkt, wobei der Schieber in dem rohrförmigen Körper (1) zwischen einer extremen hinteren Position, in welcher die vorderen (9a) und hinteren (9b) Bereiche der Kanüle (9; 9') im Inneren des rohrförmigen Körpers (1) angeordnet sind, und einer extremen vorderen Position verschiebbar ist, in welcher der vordere Bereich (9a) der Kanüle (9; 9') die Öffnung (5) der Querwand (4) des rohrförmigen Körpers (1) durchquert und über diese hinaus nach außen sichtbar vorsteht für die Entnahme einer Blutprobe und für deren Einleitung in das Vakuumröhrchen (T); und
- Sicherheitsmitteln (7, 13,14, 15a, 17) für ein definitives Feststellen des Schiebers (10) in seiner extremen hinteren Position, wenn er nach der Durchführung einer Entnahme wieder in diese Position zurückgebracht ist, wobei die Sicherheitsmittel bewegliche Organe (13, 14, 15a, 17) aufweisen, die an dem Schieber (10) angeordnet sind, und feststehende Organe (7), die an dem rohrförmigen Körper (1) angeordnet sind, **dadurch gekennzeichnet, dass** die beweglichen Organe bestehen aus:
- einem elastischen Bügel (15a), der wenigstens einen Ansatz (17, 10) aufweist, welcher zwischen einer zurückgezogenen Position, in welcher er sich vollständig im Inneren des rohrförmigen Körpers (1) befindet, und einer vorgeschobenen Position verschieblich ist, in welcher er sich wenigstens teilweise außerhalb des rohrförmigen Körpers (1) befindet und in eine seitliche Kerbe (3d) vorsteht, die in der hinteren Erweiterung (3a) des Längsschlitzes (3) vorgesehen ist, und
- einem Haken (13, 14), der zwischen einer aktiven Position, in welcher er mit dem Bügel (15a) in Eingriff ist und den Ansatz (17) in seiner zurückgezogenen Position zurückhält, und einer inaktiven Position versetzbar ist, in welcher er von dem Bügel (15a) getrennt ist und den Ansatz (17) in seine vorgeschobene Position zu kommen erlaubt, wodurch der Schieber (10) in seiner extremen hinteren Position zurückgehalten wird.

2. Vorrichtung nach Anspruch 1, **dadurch gekennzeichnet, dass** die feststehenden Organe an der Innenfläche der vorderen Querwand (4) des rohrförmigen Körpers (1) angeordnet sind.

3. Vorrichtung nach Anspruch 2, **dadurch gekennzeichnet, dass** die feststehenden Organe einen Zapfen (7) aufweisen, der für ein Versetzen des Hakens (13, 14) aus seiner aktiven Position in seine inaktive Position bestimmt ist, wenn der Schieber (10) in seine extreme vordere Position kommt.

4. Vorrichtung nach einem der Ansprüche 1 bis 3, **dadurch gekennzeichnet, dass** der Schieber (10) ein Steuerorgan (18) aufweist, welches von einem zweiten elastischen Bügel (15b) getragen wird, der hinter dem Bügel (15a) angeordnet ist, welcher den Ansatz (17) trägt, wobei das Steuerorgan durch den zweiten Bügel (15b) radial nach außen belastet ist und einen Stößel (18a) aufweist, der über den Längsschlitz (3) nach außen vorsteht, eine Fußfläche (18b), deren Größe mit derjenigen der Erweiterungen (3a, 3b) des Längsschlitzes (3) übereinstimmt, und eine Führungszone (18c), die zwischen dem Stößel (18a) und der Fußfläche (18b) angeordnet ist und deren Breite mit derjenigen des Bereichs des Längsschlitzes (3) übereinstimmt, der zwischen den vorderen (3b) und hinteren (3a) Erweiterungen angeordnet ist.

5. Vorrichtung nach Anspruch 4, **dadurch gekennzeichnet, dass** die Fußfläche (18b) zwei transversale Klauen (18e) aufweist, die gegeneinander abgebogen sind und zwischen sich eine Aufnahme ausbilden, in welcher der zweite Bügel (15b) eingeratstet ist.

6. Vorrichtung nach Anspruch 4 oder 5, **dadurch gekennzeichnet, dass** der Stößel (18a) eine Greiffläche besitzt, deren Größe für ein Überdecken der hinteren Erweiterung (3a) des Längsschlitzes (3) des rohrförmigen Körpers (1) sowie der seitlichen Kerbe (3d) dieser Erweiterung bestimmt ist, wenn sich der Schieber (10) in seiner extremen hinteren Position befindet.

7. Vorrichtung nach einem der Ansprüche 1 bis 6, **dadurch gekennzeichnet, dass** der Schieber (10) einen dritten Bügel (15c) aufweist, der vor dem den Ansatz (17) tragenden Bügel (15a) angeordnet ist und dessen äußere Abmessungen für eine nahe Verschiebemöglichkeit mit den inneren Abmessungen des rohrförmigen Körpers (1) übereinstimmen.

8. Vorrichtung nach einem der Ansprüche 1 bis 7, **dadurch gekennzeichnet, dass** die Kanüle (9) gekröpft ist, wobei ihre vorderen (9a) und hinteren (9b) Bereiche unter sich parallel sind und sich der erstere neben der Querwand (2) des rohrförmigen Körpers (1) befindet, während der zweite mit dem letzteren koaxial oder praktisch koaxial verläuft.

9. Vorrichtung nach einem der Ansprüche 1 bis 7, **dadurch gekennzeichnet, dass** die Kanüle (9') rechteckförmig ist und sich parallel zu dem rohrförmigen Körper (1) zwischen der Achse und dessen Umfang erstreckt.

10. Vorrichtung nach einem der vorhergehenden Ansprüche, **dadurch gekennzeichnet, dass** der rohrförmige Körper (1) an seinem hinteren offenen Ende einen äußeren Kragen (6) aufweist, dessen Umfang zur Ausbildung einer instabilen Abstützfläche vorgesehen ist.
